# EUROPEAN PATENT APPLICATION

(11) **EP 3 181 523 A1**
(43) Date of publication of application: **21.06.2017**
(21) Application number: 16396003.2
(22) Date of filing: 13.12.2016
(51) Int. Cl.: C02F 3/00, C02F 3/02, C02F 3/10, C02F 3/30, C05F 7/00, C02F 11/12, C02F 11/16

(54) **METHOD FOR BOOSTING BIOMASS PROCESS**

(30) Priority: 16.12.2015 FI 20150361
(71) Applicant: Esko Torkkeli, 90250 Oulu (FI)
(72) Inventor: Esko Torkkeli, 90250 Oulu (FI)

(57) **Abstract**

In a method for boosting biogas process (1) digestion residue (2A) is dried mechanically (3) and binding matter, such as dried granulated or crushed biomass, is mixed (2B, 16, 4) with it as need for starting biological drying (6) in the mixture (5) and for raising its temperature to the target range 40 - 50 °C, the mixture is fed (7) for aerobic granulation and carbonising into a drum (8) or equivalent means in which the temperature of the mixture is by means of thermophilic microbial activity raised to the range 60 - 80 °C for elevating the dry matter content of the produced granules (11) to 60 - 80 percent, and at least 50 percent of the produced granules are recirculated back to be mixed as binding matter (13) to the digestion residue and / or as input material (14) to the biogas process (1).

## Description

### FIELD OF THE INVENTION

The invention is related to a method for boosting biogas process and especially for boosting biogas process by treating and utilising digestion residue produced in the process.

### BACKGROUND OF THE INVENTION

The invention increases efficiency of the biogas reactors. Biogas may be produced from many biodegradable materials the most important of which are sewage and septic sludges, organic wastes, agricultural sludges, agricultural and forest biomasses and also waste of food and forest industries.

The most widely used biogas process is still usually a single phase continuous mesophilic (35 - 38 °C) or thermophilic (50 - 60 °C) wet process. For example sewage sludge is digested also in biphasic processes comprising first a short-term thermophilic phase and then a longer mesophilic phase in another reactor. In the wet processes the dry material content of the processed material is usually within 2 - 10 percent and at most 15 percent. Accordingly, in a normal digestion process there is a relatively small amount of both material from which gas is produced and solid material which microbes producing biogas can utilise as a substrate for forming a biofilm. Therefore gas production level is often low and digestion stations are economically not very efficient production units. Additionally, it should be taken into account that the microbes producing biogas, according to research results, are not at all able to utilise fully organic matter of the material input to a reactor but the dry matter of the digestion residue still includes 30 - 50 percent organic matter.

Digestion reactors are driven with low dry matter contents also because it is problematic to raise dry matter contents. For example mixing of the dry matter with water must be kept as good as possible in a digestion chamber so that microbial strains utilising solid matter as a substrate for growth are kept strong and affect in the whole space of the chamber. For example in Finland the dry matter content in the biggest wet processes is within 2 - 6 percent. Raising the dry matter content makes mixing more difficult and increases the quantity of energy needed for mixing. Therefore it is highly desirable that dry matter contents in the digestion reactors could be raised and substrates for growth of the digesting microbes increased so that at the same time the above mentioned problems and disadvantages of raising the dry matter content are avoided.

Also so called dry digestion plants have been developed in which mainly liquid is circulating and dissolving primarily carbonaceous matters from raw material. Carbonaceous liquid is the actual source of biogas, and solid matter works as the substrate for growth of the digesting microbes. There are also biphasic dry digestion plants where the first phase is dissolution phase and in the second phase pH is adjusted so that all carbon sources could be utilised better in biogas production.

For example biogas stations working in connection with big sewage treatment plants and having sewage sludge as principal input material produce very large amounts of digestion residue. It may be utilised primarily for soil improving and fertilising purposes. When it is post-treated by composting, large amounts of peat and long lasting composting process are needed. The market for the resulting compost product is usually unsatisfactory around big urban centres, and post-treatment costs are normally much more bigger than earnings.

Residue obtained when biogas is produced from sewage sludge is to some extent allowed to be used as such as a fertiliser but it is also restrained by many regulations. Nevertheless, the residue as such is in view of spreading, storage and properties as a fertiliser disadvantageous in comparison with many other fertiliser products.

By thermal drying and granulation it is possible to prepare marketable fertiliser products from digestion residue, and at the same time the properties of the products may be improved by adequate additives and treatments. This kind of additional treatment is however expensive, and the problem of treating reject water still remains. Therefore, for example in Finland, this alternative of treating the digestion residue has been only experimental.

FI patent application 19992178 presents a method of carbonising mainly peat but also other biomasses and at the same time possibilities to utilise the method in biogas production. Peat or other biomass is processed to pieces which are composted keeping then up efficient thermophilic microbial activity for carbonising the pieces. The temperature in composting is raised to 54 - 62 °C. Carbonised biomass obtained from the composting may be input as such or crushed to a digestion process producing biogas for increasing the amount of utilised raw material and produced gas. Also the alternative is presented in which sludge remained from the digestion process is circulated back to be mixed with the biomass to be composted.

Also many other solutions are known in which an aerobic process is combined with an anaerobic digestion process only as a part of the post treatment of the digestion residue or in a more comprehensive treatment process in which biomass is recirculated at least to some extent through an aerobic treatment back to the digestion process.

US 2015210577 A1 presents a sewage sludge digestion process in which the residue is recirculated through a kind of forced aerobic treatment in which it is heated by means of the gas produced recommendably to 60 - 70 °C feeding at the same time oxygen powerfully into the treatment reactor. The method decreases the amount of treatment residue finally produced in the process.

GB 2451519 A presents especially for waste materials including cellulose and protein a treatment solution comprising first an anaerobic and then an aerobic phase of treatment. Before the aerobic treatment phase proteinaceous biomass and specific for this purpose generated bio-accelerant, which comprises among other things sources of sugar, amino acid and inorganic salts, are added to the material under treatment. The method may comprise also recirculation to some extent in some phases.

US 7211429 B1 presents solutions in which e.g. biological waste materials are treated in the same containers both aerobically and anaerobically. There may be an aerobic section first in the treatment after which conditions in the container are changed to be suitable for anaerobic treatment. On the other hand, digestion residue may be treated e.g. by drying and other desired procedures further in the same containers, so that aerobic post-treatment is performed in this way.

Obviously the methods presented in the publications cited above are not simple enough, extensively applicable and cost-effective because they are not widely put into practice and utilised.

Accordingly, such a solution is still desirable by which the amount of digestion residue could be reduced essentially and organic matter included in the residue utilised in the biogas process itself much more better than today. The solution would also be simple enough, inexpensive to realise and serve well the development needs of bioeconomy.

### SUMMARY OF THE INVENTION

An object of the invention is to present a solution by which it is possible to essentially improve the efficiency of a digestion process and on the other hand essentially reduce the amount of digestion residue and enhance its properties in view of utilisation.

To achieve this object, a method for boosting biogas process by treating and utilising digestion residue produced in the process is characterised in that:
the digestion residue is dried mechanically and binding matter, such as dried granulated or crushed biomass, is mixed with it as need for starting biological drying in the mixture and for raising its temperature to the target range 40 - 50 °C;
the mixture is fed for aerobic granulation and carbonising into a drum or equivalent means in which the temperature of the mixture is by means of thermophilic microbial activity raised to the range 60 - 80 °C for elevating the dry matter content of the produced granules to 60 - 80 percent; and
at least 50 percent of the produced granules are recirculated back to be mixed as binding matter to the digestion residue and / or as input material to the biogas process.

Advantageously 70 - 100 percent of the produced granules are recirculated back to be mixed as binding matter to the digestion residue and / or as input material to the biogas process.

A part of the produced granules may be removed from the recirculation to be utilised in soil improving or fertilising or energy production.

### BRIEF DESCRIPTION OF THE DRAWINGS

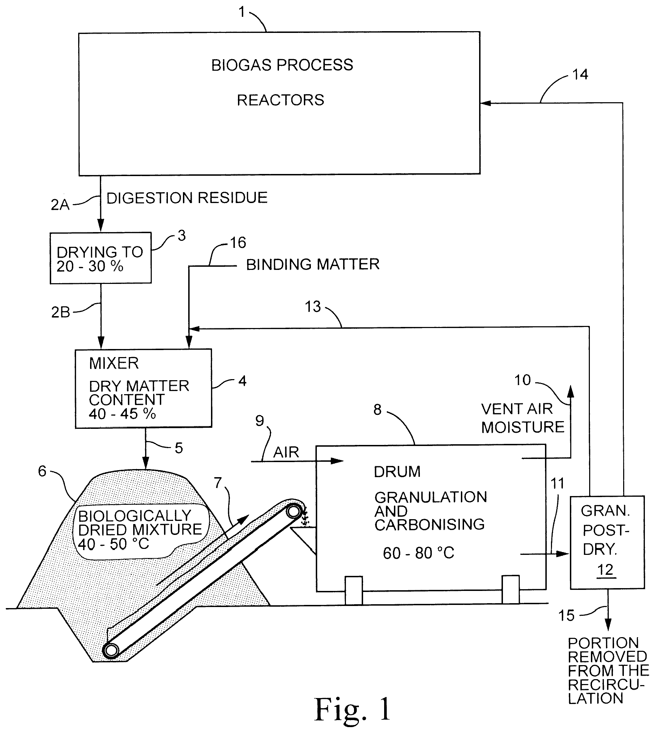

The invention and some embodiments thereof are explained in the following in further detail with reference to Fig. 1 which presents an example of the use of the method of the invention in connection with a biogas process.

### DETAILED DESCRIPTION OF THE INVENTION

In the solution according to the invention it is essential that digestion residue which still includes a lot of organic matter is treated and recirculated so that organic matter is utilised in the biogas process as well as possible and at the same time the amount of residue which finally remains from the process is reduced to a quite small fraction of the amount which it would be without the solution of the invention.

The biogas process may be wet or dry digestion process and comprise one or more digestion reactors as explained above in the section representing the background of the invention.

In Fig. 1 digestion residue 2A removed from the biogas process 1 after a production cycle is taken to be treated. The dry matter content of the residue is in wet processes normally less than 10 percent. Physical/chemical nature of the residue is low-carbonaceous high fiber mass. In phase 3 the residue is dried mechanically e.g. by pressing or centrifuging to the dry matter content 20 - 30 percent. Dried residue 2B is conducted then to a mixing phase 4 in which with it is mixed binding matter 16 a remarkable portion of which is, as indicated by arrow 13, advantageously is in this treatment process produced aerobically carbonised small-grained or crushed matter. Especially in the beginning and as need also later dried biomass from outside of the process, e.g. dried fine-grained milled peat or fine crushed sod peat, is input as binding matter.

As realised in practice, dried digestion residue 2B is conducted e.g. with a screw or belt conveyor into a mass mixer which receives in the same way also binding matters. The digestion residue and binding matters are mixed with each other carefully and the mixing is monitored and controlled so that the dry matter content of the mixture is got inside the range 40 - 45 percent.

The mixture is piled in the way indicated by arrow 5 to an intermediate storage 6 in which is started a biological drying process upholded by aerobic microbial activity, the process being controlled so that the temperature is raised relatively fast to the range 45 - 50 °C. Fast starting of the microbial activity is possible because necessary nutrients, especially liquid nitrogen, have been obtained to the mixture from the digestion residue and, on the other hand, necessary carbon compounds have been obtained from the recirculated or from outside got binding matter.

The mass which has in the intermediate storage reached the temperature of about 50 °C is fed in the way indicated by arrow 7 into a granulation and carbonising drum 8 in which the temperature is raised to the thermophilic range 60 - 80 °C and kept there by controlling air circulation, air input indicated by arrow 9 and moist air vent indicated by arrow 10. Granulating drying and carbonisation in the drum may take e.g. about 2 hours. As to the possibilities to utilise material removed from the process, it is essential that the temperature of the entire mass is kept over 70 °C at least one hour whereupon the material is hygienised and also the requirements set for hygienisation are met.

A practical realisation may be e.g. such that the drum the ends of which may be closed is filled with the mixture so that it is first about half-full. The drum is started to rotate at a speed which may be e.g. 1.5 - 2 rpm. Before starting the rotation the drum is closed and air is blown into it so that light overpressure is obtained in the drum whereby air is intruded better into the mass. The temperature is raised fast to 60 - 80 °C where it is kept in the way described above advantageously by means of automatic control. With this aerobic treatment maintained by means of thermophilic microbial activity decomposition of organic matter is achieved which in this application is called also aerobic carbonisation.

The dry matter content of the granules 11 taken out from the drum is about 60 percent, and when they are allowed to dry further in phase 12 e.g. 3 - 4 hours, the dry matter content may be raised to 70 - 80 percent. If the granules are too big, e.g. over the size of 4 mm, they can be crushed by e.g. a screw shredder or a fine shredder used in the food industries to the granular size of about 2 mm.

Dried and carbonised granules or crushed mass, in which organic matter is by means of this treatment to large extent decomposed to soluble organic carbon and to form utilisable for microbes, is circulated in the way indicated by arrows 13 and 14 both as binding matter to this drying and carbonising process of digestion residue and as input matter to the biogas process. For example 20 - 40 percent of the granules or crushed matter can be recirculated as binding matter to phase 4 in which this aerobically carbonised material is an essential agent in raising the temperature in the intermediate storing pile.

The rest of the granules or crushed matter, e. g. 60 - 80 percent, may be input back to the biogas process 1 as a solid material, nutrient for microbes, substrate for growth and source of carbon, and raw material for gas production. By means of the solution of the invention the portion of dry matter in e.g. wet process of a biogas plant may be raised to as much as 20 - 30 percent. Production of biogas in the process may be increased indeed essentially and in some cases even multiply the production.

When the dry matter content of the granules is around 60 - 80 percent they have a light hydrophobicity property which keeps them first cohesive and causes hydrophilic solid matter in flowing material to deposit on the surface thereof where digesting microbes start to create a biofilm. So the microbes utilise carbon and nutrients of the granules on the one hand for building up biofilm and on the other hand for producing biogas. As the granules are first kept cohesive they can be added to a reactor in remarkable amounts without the effect that fluency and miscibility of the material are disturbed too much. When they in the process step by step break down, available organic matter therefrom is already utilised and it is acceptable that the remained solid matter is allowed to precipitate on the bottom of the reactor to be removed then as a part of the accumulating digestion residue. The purpose of the solution according to the invention, which was to increase essentially the amount of digesting microbes and improve their action conditions as well as increase remarkably the amount of dry matter utilised in the biogas process, is then achieved.

Where appropriate, a portion of the granules may also be removed from the circulation as indicated by arrow 15. This may be necessary e.g. therefore that the amount of organic matter in the mixture is reduced too much, whereupon it may be pushed up by increasing the proportion of the binding matter brought from outside. As stated above, in this regard it is very advantageous that the granules are in the drying and carbonisation process also hygienised. E.g. those from the process removed about 4 mm granules are as such a complete carbon-phosphorus fertiliser, especially when sewage sludge is primary raw material in the biogas process. Phosphor is as such a solid material bound nutrient, and the bond is additionally strengthened with ferrochemicals. Depending on the properties of the digestion residue granules may be utilised for fertilising purposes either as such or as a base material in the production of fertiliser products. Many times also utilisation of the granulated and dried digestion residue as a fuel in energy production is acceptable and advantageous solution.

The solution of the invention may be applied also in dry digestion where it works in essentially the same way as in wet processes.

The solution of the invention is advantageous also in applications where suitable liquid material can be used for producing biogas, the liquid material being e.g. biological or other oil, sugar, alcohol, glycol or waste material such as neutralised black liquor solution which includes carbon source suitable for biogas production. A reactor may then be loaded by dried and carbonised biomass granules meant to survive certain time in the process and working primarily as a substrate for building up an as good as possible biofilm.

When desired, it is easy to combine with the aerobic process using relatively low temperature also an enzymatic treatment primarily for boosting decomposition of cellulose or hemicellulose. This may be adequate e.g. if there are a lot of field or forestry biomasses, such as straw or hay, in the materials from which biogas is produced.

The invention may vary within the scope of the accompanying claims.

## Claims

1. Method for boosting biogas process by treating and utilising digestion residue (2A) produced in the process (1), **characterised in that**:
the digestion residue (2A) is dried mechanically (3) and binding matter, such as dried granulated or crushed biomass, is mixed (2B, 16, 4) with it as need for starting biological drying (6) in the mixture (5) and for raising its temperature to the target range 40 - 50 °C;
the mixture is fed (7) for aerobic granulation and carbonising into a drum (8) or equivalent means in which the temperature of the mixture is by means of thermophilic microbial activity raised to the range 60 - 80 °C for elevating the dry matter content of the produced granules (11) to 60 - 80 percent; and
at least 50 percent of the produced granules are recirculated back to be mixed as binding matter (13) to the digestion residue and / or as input material (14) to the biogas process (1).

2. Method for boosting biogas process according to claim 1, **characterised in that** 70 - 100 percent of the produced granules are recirculated back to be mixed as binding matter (13) to the digestion residue and / or as input material (14) to the biogas process (1).

3. Method for boosting biogas process according to claim 1, **characterised in that** a part of the produced granules is removed from the recirculation to be utilised in soil improving or fertilising or energy production.
